# FASCICULE DE BREVET EUROPEEN

(11) **EP 1 851 372 B1**
(45) Date de publication et mention de la délivrance du brevet: **01.08.2018**
(21) Numéro de dépôt: 06709269.2
(22) Date de dépôt: 08.02.2006
(51) Int. Cl.: D04H 13/00, A45D 44/00, A61Q 1/14

(54) **TAMPON FIBREUX IMPRÉGNÉ**
IMPRÄGNIERTES FASERPAD
IMPREGNATED FIBROUS PAD

(30) Priorité: 14.02.2005 FR 0550424
(43) Date de publication de la demande: 07.11.2007
(73) Titulaire: Essity Operations France, 93400 Saint-Ouen (FR)
(72) Inventeur: LOUIS DIT PICARD, Bernard, F-27370 Le Bosc du Theil (FR); GREGOIRE, Philippe, F-27700 Les Andelys (FR); BRET, Bruno, F-68920 Wintzenheim (FR)
(74) Mandataire: Essity Hygiene and Health AB
(86) Numéro de dépôt international: PCT/FR2006/000281
(87) Numéro de publication internationale: WO 2006/084991

(56) Documents cités:
- EP-A- 0 681 621
- EP-A- 0 805 888
- EP-A- 0 870 496
- EP-A- 1 106 723
- WO-A-2005/009191
- FR-A- 2 655 361
- US-A1- 2003 079 323

## Description

La présente invention se rapporte au domaine des produits à usage cosmétique ou pour les soins de la peau et vise un tampon réalisé en un matériau fibreux imprégné d'une lotion à appliquer sur la peau, par exemple pour le démaquillage, les soins cosmétiques, le nettoyage de la peau, la toilette du bébé en particulier.

Pour le démaquillage, on utilise généralement un tampon de coton ou d'autre matière fibreuse, sur lequel on dépose un peu de lotion ou de lait démaquillant, puis on frotte la peau de façon à diluer ou dissoudre le maquillage et les impuretés, lesquels sont alors entraînés ou absorbés par le coton.

Les tampons que l'on désigne aussi formats sont actuellement disponibles et commercialisés sous de nombreuses formes. Ils sont découpés dans une nappe de matière fibreuse, en coton notamment, nontissée. Les tampons existent en une grande variété de tailles - de moins de 25 à plus de 100 cm²- de formes - circulaire, ovale, carrée, rectangulaire-et de grammages - de 180 à 300 g/m² environ.

Un tampon fibreux destiné à cette application doit remplir plusieurs fonctions :
- Il doit d'abord absorber la lotion ou le lait, si possible pas trop en profondeur afin qu'il reste accessible en surface.
- Il doit restituer cette lotion ou ce lait quand on le presse sur la peau, pour diluer ou dissoudre le maquillage et les impuretés.
- Il doit absorber et essuyer les impuretés et le maquillage dilué ou dissous afin de laisser la peau propre et nette.
- Il doit être suffisamment épais pour bien tenir dans la main à l'utilisation.

Le tampon doit aussi conserver son intégrité pendant l'usage. Il ne doit pas pelucher et laisser des fibres sur la peau. Il doit conserver sa forme et ne pas se désagréger, dans aucune de ses 3 dimensions. Cette caractéristique de résistance est mesurée dans ses 3 dimensions : résistance en sens marche (SM), sens de défilement de la nappe nontissée ; résistance en sens travers (ST), perpendiculaire au (SM), et force de décohésion (D), qui est la force de séparation des 2 faces du tampon, dans le sens de l'épaisseur.

On observe que par rapport à une simple nappe de coton cardé dont ils étaient constitués à l'origine, les propriétés mécaniques des tampons ont été améliorées ces dernières années par la mise en oeuvre de l'une ou l'autre des deux techniques suivantes :
Incorporation dans la masse de fibres d'un liant fusible (sous forme de fibres ou de poudre), avec chauffage par air chaud ou calandrage à chaud ; le liant agglomère les fibres de coton lors de la fusion suivie de son refroidissement et permet d'augmenter la résistance des tampons dans les 3 dimensions. Cette technique n'est cependant pas applicable pour des produits que l'on veut être composés de fibres cellulosiques seulement.

Traitement de la nappe de fibres au moyen de jets d'eau, selon un procédé d'hydroliage, qui emmêlent les fibres de surface. Ce procédé permet de réduire la propension au peluchage et d'augmenter la résistance de la nappe. Ce procédé, purement mécanique, permet de fabriquer des nappes de composition 100 % coton.

Un autre moyen connu pour se démaquiller ou nettoyer la peau consiste à utiliser des tampons déjà imprégnés d'une solution démaquillante ou de nettoyage. Ces produits sont disponibles dans le commerce et conditionnés généralement en piles dans une boite étanche, en matière plastique. Le conditionnement peut également être un sachet plastique ou un film souple soudé.

La demanderesse connaît des produits constitués d'un tissu de coton (produits tissés), de grammage 100 à 120 g/m², et imprégnés d'une solution démaquillante.

Les produits imprégnés présentent des avantages mais aussi des inconvénients par rapport à l'utilisation d'un tampon sec sur lequel on verse une lotion ou un lait.

Le tissu constituant le support des produits imprégnés est plus résistant, dans les trois dimensions, que le nontissé d'un tampon sec. En particulier, sa force de décohésion (séparation de ses 2 faces) lui est très supérieure. La force de décohésion est un paramètre important dans le cas d'un produit imprégné car les produits empilés les uns sur les autres sont adhérents et difficiles à séparer.

Cependant les techniques de filature conduisent à un produit dense dont les fibres sont compactées. Il en résulte alors une faible capacité de restitution des fluides. En effet, en raison du faible grammage et de la densité du substrat, la quantité de solution de démaquillage imprégnant chaque tampon est limitée. Un tampon en matériau tissé a donc tendance à restituer moins de lotion ou de lait par pression sur la peau, pour diluer ou dissoudre le maquillage et les impuretés, d'où une moins bonne efficacité du démaquillage ou plus généralement du nettoyage. En outre, la faible épaisseur induit une qualité perçue moindre.

On connaît WO 2005/009191 qui décrit un produit fibreux hydrolié et imprégné d'une lotion. Ce document enseigne d'une façon générale que la pression des jets doit être aussi élevée que possible. EP 870 496 décrit une feuille fibreuse hydroliée, de relativement faible grammage avec imprégnation d'une lotion.

Il existe donc un besoin parmi les tampons fibreux imprégnés pour un produit capable d'appliquer une quantité plus importante de lotion tout en conservant l'avantage du tissu quant à sa résistance mécanique.

La demanderesse s'est fixé comme objectif la réalisation d'un tampon fibreux, notamment à démaquiller, comprenant un substrat fibreux, comportant des fibres de coton, imprégné d'une lotion en particulier d'une solution démaquillante ou de nettoyage, capable de répondre à ce besoin.

Conformément à l'invention, le tampon présente les caractéristiques énumérées à la revendication 1.

L'invention a donc consisté à reconnaître que l'on pouvait réaliser l'objectif visé par l'emploi d'un matériau nontissé convenablement sélectionné de manière à présenter les caractéristiques rapportées dans la revendication principale.

Les caractéristiques de résistance mécanique du tampon selon l'invention lui permettent de conserver son intégrité et sa forme notamment lors de son prélèvement dans le conditionnement, et sa capacité de restitution, en utilisation, permet à l'utilisatrice d'appliquer une quantité de lotion, notamment démaquillante suffisante pour diluer ou dissoudre le maquillage afin de bien nettoyer la peau.

La force de décohésion est définie comme la force nécessaire pour séparer l'une de l'autre les deux faces d'un tampon. La méthode est décrite plus en détail plus loin.

Le substrat est un nontissé dont les fibres sont liées par jets d'eau selon une technique d'hydroliage désignée également aiguilletage hydraulique, connue en soi.

La demanderesse a constaté avec surprise que l'emploi d'un substrat fibreux hydrolié convenait particulièrement bien pour cette application malgré la considération que la faible force de décohésion du support ne permettait pas de garantir l'intégrité du tampon à l'usage, ni même lors du prélèvement dans le conditionnement. Le substrat présente un grammage compris entre 100 et 180 g/m² et de préférence entre 150 et 180 g/m². En outre, la résistance à la traction est au moins de 2ON en sens marche et de 10N en sens travers. La méthode de mesure de la résistance est rapportée plus loin.

De préférence, le substrat est constitué d'au moins 50 % de fibres de coton, par exemple de 100% de fibres de coton.

Il peut aussi comprendre d'autres fibres cellulosiques naturelles, comme le lin, etc... ou bien artificielles comme les fibres en viscose. Le complément peut être apporté par des fibres synthétiques, fusibles ou non.

Avantageusement, on imprègne le substrat de lotion avec un taux d'imprégnation compris entre 3 et 6, de préférence entre 4 et 5.

La lotion concernée par l'invention est de préférence à base aqueuse ; sa composition est choisie en fonction de l'usage auquel est destiné le tampon imprégné. Selon une application particulière, sa composition est adaptée pour démaquiller les yeux.

Le taux d'imprégnation est la quantité de lotion appliquée sur le substrat, rapportée à la masse de ce dernier.

Il est inférieur au taux d'absorption à saturation du substrat.

A titre d'illustration, on décrit maintenant un mode de réalisation, non limitatif, du tampon imprégné de l'invention.

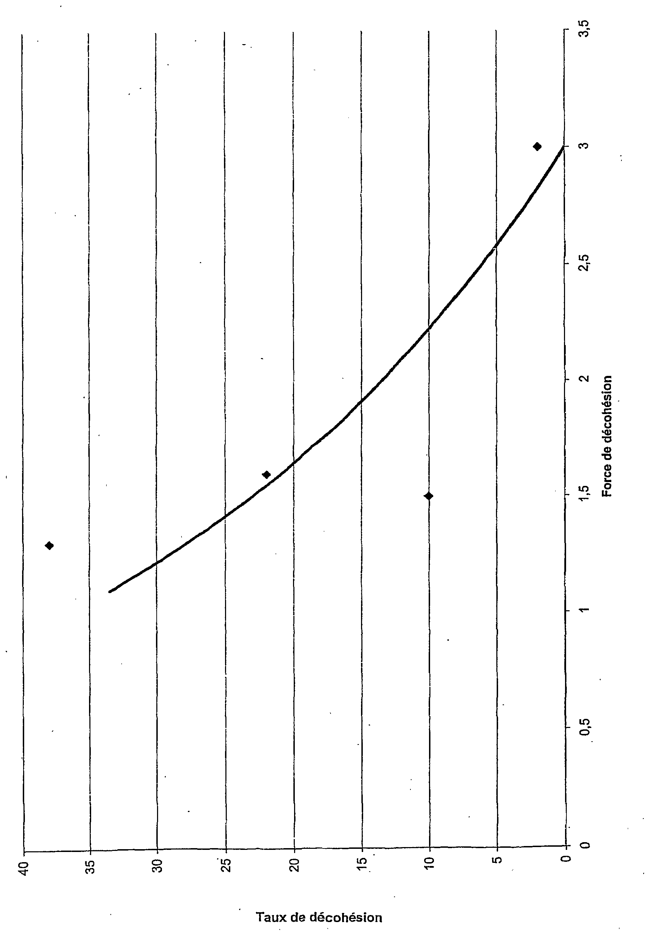

La figure unique représente le tracé de la courbe du taux de décohésion en fonction de la force de décohésion mesurés sur un tampon en nontissé de coton hydrolié.

On réalise une nappe de coton selon la technique de fabrication de nappe mentionnée dans le brevet EP 0 681 621 B1. Cette technique consiste à produire successivement et à superposer 3 couches de coton écru :
- une première couche produite par une carde, par exemple de type pêle-mêle,
- une seconde couche produite par nappage pneumatique à l'aide d'une machine de type Rando, les fibres de la couche étant orientées de manière oblique par rapport aux plans inférieur et supérieur horizontaux de la nappe, et enfin
- une troisième couche produite par une carde et similaire à la première.

Cette nappe composée de 3 couches superposées est ensuite traitée chimiquement afin de la rendre hydrophile et blanche. Selon la technique décrite dans ce brevet, la nappe ainsi formée est ensuite entraînée par un tapis support sans fin, perméable aux liquides, vers les différents postes de traitement en ligne continue. On imprègne la nappe en déversant par gravité une solution de débouillissage contenant de la soude, sur la nappe, sous la forme d'une lame liquide transversale à la direction de déplacement de celle-ci. On crée, au moyen d'une fente d'aspiration disposée sous la toile, une dépression suffisante pour permettre à au moins une partie de la solution de traverser la nappe. On contrôle en même temps la quantité de liqueur apportée à la nappe en réglant le vide créé au niveau de la fente aspirante. On introduit la nappe dans un vaporiseur chauffé à une température voisine de 100°C dans lequel elle séjourne, tout en restant continue, pendant un temps donné notamment en fonction du débit matière.

On rince ensuite la nappe, on extrait le jus de débouillissage au moyen d'une deuxième lame liquide et d'une fente à vide associée à un vide moyen.

On imprègne la nappe débouillie et hydrophile, avec une solution de blanchiment contenant de l'eau oxygénée, de la même manière que pour le traitement de débouillissage. On introduit ensuite la nappe dans un vaporiseur chauffé à une température d'environ 100°C pour que le blanchiment soit effectif.

On rince ensuite la nappe au moyen d'une succession de lames liquides associées à des fentes aspirantes.

Ce traitement de la nappe confère une adhérence entre les couches la constituant et une très bonne cohésion à l'ensemble. Cette technique permet de fabriquer des nappes de grammage compris entre 80 et 600 g/m².

Selon le mode préféré de fabrication de la nappe, on améliore la cohésion de cette dernière par un traitement de rinçage selon la technique décrite dans le brevet EP 0 805 888 B1 qui consiste à combiner le rinçage de la nappe avec l'hydroliage par de fins jets d'eau à haute pression, ce qui confère à la nappe une surface non fibreuse et de bonnes résistances mécaniques.

Les jets sont produits par des injecteurs tels qu'utilisés dans la technologie de liage hydrodynamique de nontissé. Chaque injecteur comprend, par exemple, une chambre de forme allongée, fermée sur sa longueur par une plaque perforée, en une ou plusieurs rangées, d'un grand nombre de trous de faible diamètre, de l'ordre de 80 à 200 µm. La chambre est alimentée en liquide sous pression qui s'échappe par les orifices sous forme de jets fins parallèles de diamètre correspondant. Le niveau d'énergie à fournir dépend de l'épaisseur de la nappe et de son grammage. Ce procédé permet d'obtenir un résultat très avantageux à la fois sur l'efficacité du rinçage qui est nettement améliorée par rapport à un rinçage par simple déversement de liquide et sur la consolidation de la nappe qui peut être transformée en tampon absorbant ou tampon à démaquiller par une simple découpe et un conditionnement. Ce traitement de rinçage appliqué à la nappe stratifiée, à trois couches, décrite ci-dessus présente encore l'avantage de ne pas trop réduire l'épaisseur de la nappe tout renforçant les couches superficielles.

On a réalisé des nappes, en particulier, de coton hydrophile qui se distinguaient par leur grammage : 110, 150, 180, 220, 250 et 280 g/m² respectivement mais en leur faisant subir un même traitement de rinçage différencié sur les deux faces. Des traitements différenciés sont décrits dans le brevet EP 1 106 723 B1 ou encore la demande de brevet divisionnaire EP 1 167 605 A1.

Ce traitement comprenait ici le liage d'une première face par une pluralité de jets d'eau espacés de 2,5 mm, en apportant une énergie de 1,4x10⁻³ kWh/m². Le liage de la deuxième face était réalisé au moyen d'une pluralité de jets d'eau espacés de 0,6 mm, apportant une énergie de 0,9x10⁻³ kWh/m². Après liage la nappe était séchée et découpée en tampons au format souhaité par des moyens appropriés.

Afin de déterminer dans quelle mesure des tampons fabriqués suivant ce type de procédé sont susceptibles d'être aptes à un usage comme tampon imprégné de lotion de démaquillage, on a procédé à des essais sur des tampons imprégnés d'une lotion de démaquillage.

La composition de la lotion d'imprégnation utilisée correspond à celle d'une lotion de démaquillage. Elle est aqueuse. A titre d'exemple, cette lotion peut comprendre les composants suivants : eau, paraffine liquide, butylène glycol, isohexadécane, palmitate d'isopropyle, cyclométhicone, stéarate de glycéryle, panthénol, cétéareth-20, cétéareth-12, alcool cétéarylique, palmitate de cétyle, propylène glycol, disodium EDTA, sodium hydroxide, parfum et conservateurs.

### Taux d'imprégnation

Le taux d'imprégnation est le rapport entre la quantité de liquide en poids appliquée sur le tampon et le poids du tampon sec. Ce taux est inférieur au taux d'absorption à saturation. On mesure ce dernier à partir d'un test d'absorption de la pharmacopée européenne.

### Mesure de l'absorption à saturation

On utilise un panier cylindrique, préalablement séché, constitué par des fils de cuivre d'un diamètre de 0,4 mm environ. Ce panier a une hauteur de 8,0 cm, un diamètre de 5,0 cm et des mailles d'une largeur de 1,5 à 2 cm. Sa masse est de 2,7 +/- 0,3 g.

Le test a été réalisé à partir de disques de 57 mm de diamètre (tampons en non-tissé) ou de 50 mm de diamètre (produit du marché en tissu).

Le disque est préalablement pesé sec sur une balance à 0,01 g près. On obtient le poids M1. Le disque est placé dans le panier. Il est incurvé tangentiellement à la courbure du panier. On pèse l'ensemble du panier et du disque sec à 0,01 g près. On obtient le poids M2. On prépare un récipient de 11 à 12 cm de diamètre rempli d'eau à 20° environ sur une hauteur de 10 cm. Le panier et le disque sont immergés dans l'eau puis sont égouttés en tenant le panier horizontalement hors de l'eau pendant 30 s.

On pèse alors l'ensemble du panier et du disque saturé, à 0,01 g près. On obtient le poids M3.

L'absorption à saturation par tampon est déterminée par la différence M3-M2. Le taux d'absorption à saturation est déterminé par le rapport : (M3-M2)/M1. Le taux d'imprégnation choisi pour les formats de l'invention est nécessairement inférieur à ce taux d'absorption à saturation.

Par exemple, pour les échantillons de produits rapportés plus loin, on a mesuré les valeurs d'absorption à saturation.

| | 1 | 2 | 3 | 4 | Tissu 120 g/m² |
|---|---|---|---|---|---|
| Poids de format (g) | 0,46 | 0,46 | 0,46 | 0,46 | 0,24 |
| Absorption à saturation (g) | 6,44 | 6,39 | 6,03 | 7,45 | 2,33 |
| Taux d'absorption à saturation (g/g) | 14,0 | 13,9 | 13,1 | 16,2 | 9,7 |

### Test de décohésion (Mesure du taux de décohésion)

On a réalisé six séries de disques, chacune correspondant à un grammage de nappe. Le diamètre des disques découpés était de 57 mm. On a choisi un taux d'imprégnation des disques de 4,5. On a conditionné les disques dans une boîte en plastique cylindrique à raison de 30 disques par boîte.

Le test de préhension dans la boîte a consisté à prélever manuellement les disques un à un, ainsi que peut le faire une utilisatrice. On a contrôlé s'ils conservaient leur intégrité durant cette opération. En effet, en raison de leur imprégnation, les tampons ont tendance à coller les uns aux autres. Par ailleurs, un produit hydrolié de grammage élevé, n'est pas homogène dans son épaisseur ; les couches centrales sont moins liées que les couches superficielles. Il s'ensuit un risque de délaminage certain. On a déterminé un taux de délaminage ou de décohésion pour chaque série de tampons imprégnés. Ce taux correspond au nombre de tampons imprégnés qui ont été délaminés rapporté à cent.

### Force de décohésion

On a mesuré par ailleurs la force de décohésion de chacune des nappes.

La force de décohésion est mesurée de la façon suivante :
On utilise un dynamomètre, ici Adamel Lhomargy DY 30 (n°61060 de juillet 1995), muni d'un capteur 100N.

On place un disque échantillon entre deux plateaux solidaires chacun d'un bras du dynamomètre. Le disque est maintenu au moyen de rubans adhésifs double face (dimensions 60x60 mm pour les tampons de 57 mm de diamètre). On presse les plateaux l'un contre l'autre à 7N. On tire ensuite à 100 mm/min et on relève la valeur maximale de la force de décohésion jusqu'à la séparation des plateaux. La force de décohésion retenue est la moyenne de cette force mesurée sur cinq échantillons de même type.

On a reporté dans le tableau ci-dessous le taux de décohésion pour les tampons imprégnés et la force de décohésion du substrat sec.

| | Tissu de coton | Nontissé coton 110 g/m² | Nontissé coton 150 g/m² | Nontissé coton 180 g/m² | Nontissé coton 220 g/m² | Nontissé coton 250 g/m² | Nontissé coton 280 g/m² |
|---|---|---|---|---|---|---|---|
| Taux de décohésion | 0 | 0 | 0 | 2% | 10 % | 22% | 38 % |
| Force de décohésion | Non mesurable | Non mesurable | 3,83 N | 2,98 N | 1,48 N | 1,53 N | 1,35 N |

On vérifie sur ce tableau que la force de décohésion mesurée sur le substrat sec décroît dans la gamme 150-280 g/m².

Le taux de décohésion contrôlé lors du prélèvement dans le conditionnement croît avec le grammage.

On a tracé la courbe du taux de décohésion en fonction de la force de décohésion du substrat sec. Cette courbe est représentée en annexe.

On cherche le plus haut grammage possible compte tenu du taux de décohésion acceptable, en vue d'un produit présentant une qualité perçue optimale. Le grammage maximum acceptable est ainsi de 180 g/m², le taux de délaminage de 2 % étant satisfaisant. On retient en fait une valeur intermédiaire de 5% de taux de décohésion en deçà duquel le produit est satisfaisant. Cette valeur correspond à une force de décohésion d'environ 2,5N.

A partir de ce grammage de 180 g/m², on cherche à optimiser l'énergie d'hydroliage afin d'obtenir le meilleur compromis entre la cohésion de nappe, l'épaisseur, l'absorption et le pouvoir de restitution de solution démaquillante d'imprégnation. On sait en effet que plus l'apport en énergie hydraulique est important, plus on augmente la cohésion de nappe, la conséquence étant une perte d'épaisseur et d'absorption.

Une nappe de 180 g/m² a donc été traitée selon 4 recettes d'hydroliage différentes :
1) Traitement d'une première face par une pluralité de jets d'eau espacés de 2,5 mm, tel que décrit dans le brevet EP 1 106 723 B1, apportant une énergie de 5,71 x 10⁻³ kWh/m² ; traitement de la deuxième face par une pluralité de jets d'eau espacés de 0,6 mm, apportant une énergie de 2,08 x 10⁻³ kWh/m², tel que décrit ci-dessus.
2) Traitement d'une première face par une pluralité de jets d'eau espacés de 2,5 mm, tel que décrit dans le brevet EP 1 106 723 B1, apportant une énergie de 3,75 x 10⁻³ kWh/m² ; traitement de la deuxième face par une pluralité de jets d'eau espacés de 0,6 mm, apportant une énergie de 2,08 x 10⁻³ kWh/m².
3) Traitement d'une première face par une pluralité de jets d'eau espacés de 2,5 mm, tel que décrit dans le brevet EP 1106 723 B1, apportant une énergie de 8,04 x 10⁻³ kWh/m² ; traitement de la deuxième face par une pluralité de jets d'eau espacés de 0,6 mm, apportant une énergie de 2,08 x 10⁻³ kWh/m².
4) Traitement d'une première face par une pluralité de jets d'eau espacés de 0,6 mm, apportant une énergie de 5,71 x 10⁻³ kWh/m² ; traitement de la deuxième face par une pluralité de jets d'eau espacés de 0,6 mm, apportant une énergie de 2,08 x 10⁻³ kWh/m². Les deux faces ne sont pas différenciées.

Par rapport à la première recette testée pour déterminer le grammage optimum, l'énergie sur la première face a été diminuée pour l'essai 2, augmentée pour l'essai 3. L'espacement des jets de la première face est le même que sur la deuxième face pour l'essai 4.

A partir de ces nappes, des échantillons ont été réalisés comme dans le test précédent. Les disques ont un diamètre de 57 mm. Ils ont été imprégnés de la solution démaquillante décrite plus haut, au taux d'imprégnation de 4,5.

Les principales caractéristiques des disques ont été mesurées sur les disques secs d'une part et sur les disques imprégnés d'autre part.

Sur les disques secs on a mesuré l'épaisseur, la résistance à la traction et la résistance à la décohésion ou force de décohésion.

L'épaisseur est mesurée selon la norme officielle « Edana 30-5-99 for normal nonwovens », voir le paragraphe 5-2 de la méthode présentée dans cette norme. La mesure a été effectuée sous une pression de 0,5 kPa.

Les résistances à la traction SM et ST sont mesurées de la façon suivante :
- On découpe dans le tampon un échantillon de 25 mm de large respectivement dans le sens marche et dans le sens travers. Les tampons ont été préalablement conservés pendant 24 heures dans une atmosphère à 65% d'hygrométrie +/- 2% à 20°C +/- 2%.
- On place l'échantillon entre les mâchoires d'un dynamomètre (ici le même que précédemment). Les mâchoires sont écartées de 30 mm. Elles sont plus larges que l'échantillon. On déclenche le dynamomètre, la vitesse étant de 100 mm/min.
- On relève la valeur maximale jusqu'à la rupture de l'échantillon. Les résistances à la traction retenues sont la moyenne des mesures effectuées sur cinq échantillons.

La résistance à la décohésion ou force de décohésion est mesurée comme précédemment.
- Sur les disques imprégnés, on a mesuré la quantité de solution de démaquillage restituée sous pression.

La méthode de mesure de la quantité de solution restituée sous pression consiste à imprégner les tampons au taux défini, soit ici 4,5 grammes de lotion par gramme de coton. On a vérifié que ce taux était inférieur au taux d'absorption à saturation. Puis on contrôle la quantité restituée par application d'une charge sur le tampon.

Le mode opératoire de la mesure de la quantité restituée sous pression est décrit en détail ci-après.
A/ Objet : Mesure de la quantité de lotion pouvant être libérée sous pression par un tampon imprégné.
   On a déterminé préalablement à l'aide d'une balance que la pression moyenne exercée par les doigts sur le visage lors de l'opération de démaquillage était de l'ordre de 0,3 kg /cm². On adapte donc la charge en fonction de la surface du tampon à tester de façon à tester toujours avec cette pression.
B/ Application de la charge.
   1/ Peser le tampon imprégné de lotion à l'aide d'une balance à 0,01 g près : poids M1.
   2/ Préparer du papier buvard (Schleisser & Schull n° 860) découpé (diamètre 112 mm ou carré de 145 mm de côté) avec l'emporte-pièce ou au ciseau (on utilise 10 couches de buvard afin d'obtenir une bonne capacité d'absorption).
   3/ Poser le tampon imprégné de lotion sur les 5 couches inférieures de buvard et mettre 5 couches de buvard dessus.
   4/ Poser la plaque blanche sur le sandwich (buvards + format + buvards), afin que la répartition de la charge soit correcte.
   5/ Appliquer la charge durant 1 minute.
      Le poids est de 7,65 kg pour 0,00255 m² (disque de diamètre 57 mm). Celui-ci est calculé en fonction de la surface du tampon.
      Exemple : Si la surface d'un tampon est de 0,0100 m², alors la charge à appliquer est de = 30kg.
   6/ Retrait de la charge ; pesée du tampon. On obtient la quantité de lotion extraite en calculant la différence entre le poids du tampon après compression et le poids du tampon avec lotion avant compression.

On a procédé aux mêmes mesures sur un tampon imprégné disponible dans le commerce, dont le substrat est un tissu, gratté deux faces, de grammage 120 g/m².

Les résultats sont rapportés sur les tableaux ci-dessous.

On constate que le substrat en nontissé permet de restituer une quantité de lotion plus importante que le tissu.

**Disques secs.**

| | 1 | | 2 | 3 | 4 | Tissu 120 g/m² |
|---|---|---|---|---|---|---|
| Poids du tampon (g) | 0,46 | | 0,46 | 0,46 | 0,46 | 0,24 |
| Epaisseur (mm) | 2,00 | | 2,06 | 1,80 | 2,00 | 0,88 |
| Absorption à saturation (g) | 6,44 | | 6,39 | 6,03 | 7,45 | 2,33 |
| Taux d'absorption à saturation (g/g) | 14,0 | | 13,9 | 13,1 | 16,2 | 9,7 |
| Résistance à la rupture (N) | SM | 25,2 | 19,0 | 44,4 | 37,4 | > 100 |
| | ST | 13,3 | 8,5 | 26,0 | 15,8 | > 100 |
| Force de décohésion (N) | 2,98 | | 1,18 | 6,87 | 1,97 | Non mesurable |

**Disques imprégnés.**

| | 1 | 2 | 3 | 4 | Tissu 120 g/m² |
|---|---|---|---|---|---|
| Quantité restituée en gftampon | 0,79 | 0,78 | 0,92 | 0,74 | 0,42 |
| Quantité restituée en g/m² (*) | 310 | 306 | 361 | 290 | 214 |

| | | | | | |
|---|---|---|---|---|---|
| (*) g de lotion par m² de substrat | | | | | |

On observe que l'échantillon 2 présente une résistance à la décohésion faible en raison de l'application d'une énergie d'hydroliage insuffisante et que l'échantillon 4 présente le même défaut en raison de la non différenciation comparativement à la différentiation décrite dans le brevet EP 1 106 723 B1 mentionné plus haut. Les échantillons 1 et 3 bénéficient d'un meilleur compromis entre les différentes contraintes : quantité restituée, résistance à la décohésion et qualité perçue. En raison du critère de qualité perçue liée à la « main » du tampon (la main est la sensation de consistance que donne une étoffe lorsqu'on la saisit manuellement), on détermine que l'épaisseur minimale est d'au moins 1,8 mm pour ces échantillons. Cependant, l'échantillon 1 est préféré au 3 en raison de son épaisseur plus élevée.

## Revendications

1. Tampon fibreux imprégné pour le soin de la peau comprenant un substrat fibreux, comportant des fibres de coton, imprégné d'une lotion à appliquer sur la peau, le substrat étant un nontissé lié par jets d'eau **caractérisé en ce que** ledit substrat nontissé présente un grammage compris entre 100 et 180 g/m², de préférence entre 150 et 180 g/m² présentant la capacité de restituer sous pression au moins 250 g de lotion par m² de substrat, le substrat à sec ayant une force de décohésion d'au moins 2,5 N et une épaisseur d'au moins 1,8 mm.

2. Tampon selon la revendication 1 dont le substrat est constitué d'au moins 50% de fibres de coton.

3. Tampon selon la revendication précédente dont le substrat est constitué de 100 % de fibres de coton.

4. Tampon selon la revendication 1 dont le taux d'imprégnation de la lotion est compris entre 3 et 6, de préférence entre 4 et 5.

5. Tampon selon la revendication 1 dont la lotion est à usage démaquillant.

6. Tampon selon l'une des revendications précédentes dont l'épaisseur est 1,8 mm.

7. Tampon selon la revendication 1 dont la résistance mécanique du substrat est au moins de 10 N en ST.

8. Tampon selon la revendication précédente dont la résistance à la traction du substrat est au moins de 20 N en SM.

## Patentansprüche

1. Imprägniertes Faserpad zur Hautpflege, umfassend ein Fasersubstrat, umfassend Baumwollfasern, das mit einer Lotion zum Auftragen auf die Haut imprägniert ist, wobei das Substrat ein wasserstrahlverfestigtes Vlies ist, **dadurch gekennzeichnet, dass** das Vliessubstrat ein Flächengewicht im Bereich zwischen 100 und 180 g/m², vorzugsweise zwischen 150 und 180 g/m² aufweist, das die Fähigkeit aufweist, unter Druck mindestens 250 g Lotion pro m² Substrat abzugeben, wobei das Trockensubstrat eine Dekohäsionskraft von mindestens 2,5 N und eine Dicke von mindestens 1,8 mm aufweist.

2. Pad nach Anspruch 1, wobei das Substrat zu mindestens 50 % aus Baumwollfasern besteht.

3. Pad nach dem vorhergehenden Anspruch, wobei das Substrat zu 100 % aus Baumwollfasern besteht.

4. Pad nach Anspruch 1, wobei die Imprägnierungsrate der Lotion im Bereich zwischen 3 und 6, vorzugsweise zwischen 4 und 5 liegt.

5. Pad nach Anspruch 1, wobei die Lotion zur Verwendung als Reinigungsmittel vorgesehen ist.

6. Pad nach einem der vorhergehenden Ansprüche, dessen Dicke 1,8 mm beträgt.

7. Pad nach Anspruch 1, wobei die mechanische Festigkeit des Substrats mindestens 10 N in Querrichtung beträgt.

8. Pad nach dem vorhergehenden Anspruch, wobei die Zugfestigkeit des Substrats mindestens 20 N in Laufrichtung beträgt.

## Claims

1. Impregnated fibrous pad for caring for the skin comprising a fibrous substrate, comprising cotton fibres, impregnated with a lotion to be applied to the skin, the substrate being a nonwoven entangled by jets of water, **characterized in that** the said nonwoven substrate exhibits a basis weight of between 100 and 180 g/m², preferably between 150 and 180 g/m², exhibiting the ability to restore, under pressure, at least 250 g of lotion per m² of substrate, the dry substrate having a debonding force of at least 2.5 N and a thickness of at least 1.8 mm.

2. Pad according to Claim 1, the substrate of which is composed of at least 50% of cotton fibres.

3. Pad according to the preceding claim, the substrate of which is composed of 100% of cotton fibres.

4. Pad according to Claim 1, the degree of impregnation of which with the lotion is between 3 and 6, preferably between 4 and 5.

5. Pad according to Claim 1, the lotion of which is for make-up-removing use.

6. Pad according to one of the preceding claims, the thickness of which is 1.8 mm.

7. Pad according to Claim 1, the mechanical strength of the substrate of which is at least 10 N in CD.

8. Pad according to the preceding claim, the tensile strength of the substrate of which is at least 20 N in MD.
